**Europäisches Patentamt**

**European Patent Office**

**Office européen des brevets**

(19)

(11) Veröffentlichungsnummer : **0 491 196 B1**

(12)

# EUROPÄISCHE PATENTSCHRIFT

(45) Veröffentlichungstag der Patentschrift :
**04.08.93 Patentblatt 93/31**

(51) Int. Cl.⁵ : **C10G 7/08,** C07C 7/08

(21) Anmeldenummer : **91120384.2**

(22) Anmeldetag : **28.11.91**

(54) **Verfahren zur Abtrennung von Aromaten aus Kohlenwasserstoffgemischen mit beliebigem Aromatengehalt.**

(30) Priorität : **15.12.90 DE 4040145**

(43) Veröffentlichungstag der Anmeldung :
**24.06.92 Patentblatt 92/26**

(45) Bekanntmachung des Hinweises auf die
Patenterteilung :
**04.08.93 Patentblatt 93/31**

(84) Benannte Vertragsstaaten :
**BE DE FR GB IT NL**

(56) Entgegenhaltungen :
**DE-A- 1 803 758**
**DE-A- 3 409 030**
**FR-A- 2 173 011**
**FR-A- 2 272 055**

(73) Patentinhaber : **Krupp Koppers GmbH**
**Altendorfer Strasse 120**
**W-4300 Essen 1 (DE)**

(72) Erfinder : **Skatulla, Luzian**
**Theodor-Suhnel-Strasse 14**
**W-4330 Mülheim/Ruhr (DE)**
Erfinder : **Schneider, Hans-Christoph**
**Rosental 8**
**W-4320 Hattingen (DE)**
Erfinder : **Vollmer, Hans-Jürgen, Dr.**
**Dahlienhof 13**
**W-4300 Essen (DE)**

EP 0 491 196 B1

## Beschreibung

Verfahren zur Abtrennung von Aromaten aus Kohlenwasserstoffgemischen beliebigen Aromatengehaltes.

Die Erfindung betrifft ein Verfahren zur Abtrennung von Aromaten aus Kohlenwasserstoffgemischen beliebigen Aromatengehaltes, die als nichtaromatische Bestandteile insbesondere Paraffine, Cycloparaffine, Olefine, Diolefine sowie organische Schwefelverbindungen enthalten können, durch Extraktivdestillation, bei der N-substituierte Morpholine, deren Substituenten nicht mehr als sieben C-Atome aufweisen, als selektives Lösungsmittel verwendet werden, wobei die nichtaromatischen Bestandteile des als Einsatzprodukt dienenden Kohlenwasserstoffgemisches als Raffinat über Kopf aus der Extraktivdestillationskolonne abdestilliert werden, während die Aromaten zusammen mit dem verwendeten Lösungsmittel als Extrakt aus dem Sumpf der Extraktivdestillationskolonne abgezogen und anschließend in einer nachgeschalteten Abtreiberkolonne destillativ vom Lösungsmittel abgetrennt werden.

Das vorstehend beschriebene Aromatengewinnungsverfahren ist bereits seit einer ganzen Reihe von Jahren bekannt und hat sich, insbesondere unter Verwendung von N-Formylmorpholin als selektivem Lösungsmittel, in der Zwischenzeit in der Praxis auf verschiedenen großtechnischen Anlagen gut bewährt. Hierbei wird normalerweise das aus dem Sumpf der Abtreiberkolonne abgezogene Lösungsmittel nach entsprechender Abkühlung zur Wiederverwendung in die Extraktivdestillationskolonne zurückgeführt, wobei aus verfahrenstechnischen Gründen die Wiedereinleitung des Lösungsmittels bisher am Kopf der Extraktivdestillationskolonne erfolgte. Dadurch läßt es sich aber praktisch nicht ververmeiden, daß das anfallende Raffinat noch gewisse Lösungsmittelreste enthält, wobei der Lösungsmittelgehalt im Raffinat bis zu 2 Gew.-% betragen kann. Aus Wirtschaftlichkeitsgründen und im Hinblick auf die Gewinnung eines möglichst reinen Raffinates ist es jedoch unerläßlich, diesen Lösungsmittelanteil im Raffinat möglichst weitgehend zurückzugewinnen.

Bisher war es deshalb üblich, das als Kopfprodukt aus der Extraktivdestillationskolonne abgezogene Raffinat in eine separate Destillationskolonne einzuleiten, in der die Kohlenwasserstoffe des Raffinates vom Lösungsmittel destillativ abgetrennt wurden. Da die hierbei als Kopfprodukt gewonnenen Kohlenwasserstoffe des Raffinates in der Praxis meist nur noch einen Lösungsmittelgehalt von < 1 ppm aufweisen dürften, erfordert diese destillative Trennung allerdings einen hohen apparativen Aufwand (Destillationskolonne mit hoher Bodenzahl) und einen hohen Energieverbrauch.

Um diesen hohen Energieverbrauch herabzusetzen, ist deshalb in der DE-OS 34 09 030 bereits ein Verfahren der gattungsgemäßen Art vorgeschlagen worden, bei dem die Destillation des als Kopfprodukt der Extraktivdestillation anfallenden Raffinates unter solchen Bedingungen betrieben wird, daß das dabei anfallende Sumpfprodukt noch einen Lösungsmittelgehalt von 20 bis 75 Gew.-% aufweist. Anschließend wird dieses Sumpfprodukt in einem Scheidebehälter in eine schwere und eine leichte Phase getrennt, wobei die lösungsmittelreiche schwere Phase in die Extraktivdestillationskolonne und die lösungsmittelarme leichte Phase in die Raffinatdestillationskolonne zurückgeführt wird. Mit dieser Methode gelingt es zwar, den Energiebedarf für die Nachreinigung des Raffinates herabzusetzen. Es ist jedoch immer noch eine separate Kolonne für die Destillation des Raffinates sowie zusätzlich ein Scheidebehälter für die Trennung von schwerer und leichter Phase erforderlich, was einen nicht unbeträchtlichen apparativen Aufwand bedeutet.

Der Erfindung liegt deshalb die Aufgabe zugrunde, bei einem Verfahren der gattungsgemäßen Art den für die Nachbehandlung des Raffinates erforderlichen apparativen Aufwand weiter zu senken, ohne daß hierbei ein zusätzlicher Energiebedarf entsteht. Gleichzeitig soll dabei die Reinheit des gewonnenen Raffinates und Extraktes nicht beeinträchtigt, sondern möglichst noch verbessert werden.

Diese Aufgabe wird erfindungsgemäß dadurch gelöst, daß sowohl die Extraktivdestillation als auch die Entfernung der Lösungsmittelreste aus dem Raffinat in einer einzigen gemeinsamen Kolonne durchgeführt werden, die oberhalb der Lösungsmittelaufgabe einen zusätzlichen Kolonnenteil aufweist, wobei das Einsatzkohlenwasserstoffgemisch vor dem Eintritt in diese Kolonne im indirekten Wärmeaustausch mit dem von der Abtreiberkolonne kommenden heißen Lösungsmittel bis auf eine Temperatur zwischen 130 und 150°C erhitzt wird.

Gemäß einer bevorzugten Ausführungsform des erfindungsgemäßen Verfahrens wird dabei das erhitzte Einsatzkohlenwasserstoffgemisch vor der Einleitung in die Extraktivdestillationskolonne in einem Trennbehälter entspannt und dadurch in eine flüssige und eine dampfförmige Phase zerlegt, die getrennt voneinander in die Extraktivdestillationskolonne eingeleitet werden. Die dampfförmige Phase wird hierbei unterhalb der Aufgabestelle für die flüssige Phase in die Extraktivdestillationskolonne eingeleitet.

Die Lage der Aufgabestellen wird in Abhängigkeit von der Zusammensetzung des Einsatzkohlenwasserstoffgemisches ermittelt.

Die zur Durchführung des erfindungsgemäßen Verfahrens geeignete Extraktivdestillationskolonne weist oberhalb der Lösungsmittelaufgabe einen zusätzlichen Kolonnenteil auf, in dem die Entfernung der Lösungsmittelreste aus dem Raffinat erfolgt. Dieser Kolonnenteil kann mit Böden oder sonstigen Einbauten versehen

2

sein, wobei die Bodenzahl jedoch vorzugsweise nicht höher sein soll als die Bodenzahl, die in einer separaten Raffinatdestillationskolonne erforderlich wäre.

Daß mit dem erfindungsgemäßen Verfahren das anstehende Problem erfolgreich gelöst werden kann, ist insofern überraschend, als man bisher immer davon ausgegangen ist, daß die Entfernung der Lösungsmittelreste aus dem Raffinat in einer separaten Kolonne erfolgen müsse, um einen Raffinatrückfluß in der Extraktivdestillationskolonne möglichst weitgehend zu vermeiden. Hierfür waren folgende Überlegungen maßgebend:

1. Ein Raffinatrückfluß führt zu einer Verdünnung des Lösungsmittels und damit zu einer Selektivitätsminderung, wodurch die gewünschte Stofftrennung unnötig erschwert werden könnte.

2. Hochselektive Lösungsmittel - und hierzu gehören die eingangs genannten N-substituierten Morpholine - weisen nur ein begrenztes Lösevermögen für die abzutrennenden nichtaromatischen Kohlenwasserstoffe auf. Ein Raffinatrückfluß könnte deshalb unter Umständen dazu führen, daß sich auf den oberen Böden der Extraktivdestillationskolonne zwei Flüssigphasen mit unterschiedlichen Dichten ausbilden, die einen störungsfreien Betrieb der Extraktivdestillationskolonne unmöglich machen könnten.

Die praktischen Erfahrungen bei der Anwendung des erfindungsgemäßen Verfahrens haben jedoch gezeigt, daß diese Befürchtungen nicht gerechtfertigt waren.

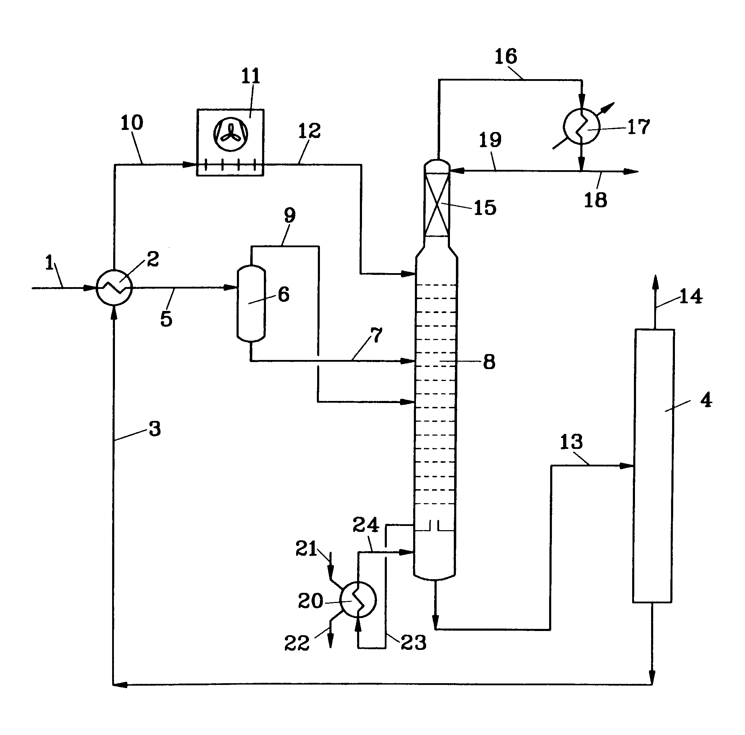

Weitere Einzelheiten des erfindungsgemäßen Verfahrens sollen an Hand des in der Abbildung dargestellten Fließschemas erläutert werden. Das Fließschema enthält dabei nur die für die Verfahrenserläuterung unbedingt notwendigen Anlagenteile, während Nebeneinrichtungen, wie beispielsweise Pumpen, Umlaufkocher, Wärmetauscher, Meß- und Regeleinrichtungen etc., nicht dargestellt sind.

Bei dem in der Abbildung dargestellten Fließschema wird das Einsatzkohlenwasserstoffgemisch über die Leitung 1 in den Wärmetauscher 2 eingeleitet und dort im indirekten Wärmeaustausch mit dem von der Abtreiberkolonne 4 kommenden heißen Lösungsmittel, das über die Leitung 3 zugeführt wird, bis auf eine Temperatur zwischen 130 und 150°C erhitzt. Bei dem im Fließschema dargestellten Ausführungsbeispiel wird das erhitzte Einsatzkohlenwasserstoffgemisch anschließend über die Leitung 5 in den Trennbehälter 6 eingeleitet, in dem es durch Entspannung in eine flüssige und eine dampfförmige Phase zerlegt wird. Die flüssige Phase wird hierbei über die Leitung 7 in den mittleren Teil der mit Böden versehenen Extraktivdestillationskolonne 8 eingeleitet. Beispielsweise kann die Einleitung bei einer Gesamtbodenzahl der Kolonne von 55 Böden in Höhe des 24. Bodens von oben erfolgen. Gleichzeitig wird die dampfförmige Phase über die Leitung 9 unterhalb der Aufgabestelle für die flüssige Phase in die Extraktivdestillationskolonne 8 eingeleitet. Im vorliegenden Falle kann die Einleitung der dampfförmigen Phase beispielsweise 6 Böden unterhalb der Aufgabestelle für die flüssige Phase erfolgen. Wie bereits weiter oben erwähnt wurde, kann das erfindungsgemäße Verfahren gegebenenfalls auch in der Weise durchgeführt werden, daß das vom Wärmetauscher 2 kommende heiße Einsatzkohlenwasserstoffgemisch über die Leitung 5 unmittelbar in den mittleren Teil der Extraktivdestillationskolonne 8 eingeleitet wird.

In dieser Kolonne erfolgt die Auftrennung des Einsatzkohlenwasserstoffgemisches unter dem Einfluß des Lösungsmittels in an sich bekannter Weise. Das vom Wärmetauscher 2 kommende Lösungsmittel gelangt hierbei über die Leitung 10 in den Luftkühler 11, in dem es gegebenenfalls die notwendige Abkühlung erfährt, um anschließend mit einer Temperatur zwischen 100 und 110°C über die Leitung 12 in die Extraktivdestillationskolonne 8 eingeleitet zu werden. Das eingeleitete Lösungsmittel fließt über die Böden dieser Kolonne nach unten herab, wobei es die dampfförmigen Aromaten aufnimmt. Das flüssige Sumpfprodukt, das aus dem Lösungsmittel und den darin gelösten Aromaten besteht, wird über die Leitung 13 aus der Extraktivdestillationskolonne 8 abgezogen und in die Abtreiberkolonne 4 eingeleitet, in der dieses vielfach auch als Extrakt bezeichnete Sumpfprodukt in seine Bestandteile zerlegt wird. Auf konstruktive Einzelheiten der Abtreiberkolonne 4 braucht hier nicht näher eingegangen zu werden, da die Aufarbeitung des Extraktes der Extraktivdestillation nicht Gegenstand der vorliegenden Erfindung ist. Die Aromaten werden als Kopfprodukt aus der Abtreiberkolonne 4 über die Leitung 14 abgezogen, während sich das aromatenfreie Lösungsmittel im Sumpf dieser Kolonne sammelt und über die Leitung 3 dem Wärmetauscher 2 zugeführt werden kann.

Die nichtaromatischen Kohlenwasserstoffe des Einsatzkohlenwasserstoffgemisches, die die Raffinatphase bilden, steigen währenddessen in der Extraktivdestillationskolonne 8 dampfförmig nach oben. Damit aus diesen nichtaromatischen Kohlenwasserstoffen die Lösungsmittelreste entfernt werden können, weist die Extraktivdestillationskolonne 8 oberhalb der Lösungsmittelaufgabe, d.h oberhalb der Einmündung der Leitung 12 in diese Kolonne, einen zusätzlichen Kolonnenteil 15 auf. Dieser Kolonnenteil 15, der mit Böden oder sonstigen Einbauten versehen sein kann, bildet zusammen mit der Extraktivdestillationskolonne 8 eine bauliche Einheit. In der vorliegenden Abbildung weist der Kolonnenteil 15 einen etwas geringeren Durchmesser auf als die übrige Extraktivdestillationskolonne 8. In der Praxis können aber beide Teile auch den gleichen Durchmesser besitzen. Die von den Lösungsmittelresten befreiten nichtaromatischen Kohlenwasserstoffe entweichen dampfförmig über Kopf aus dem Kolonnenteil 15 und gelangen über die Leitung 16 in den Kühler 17, in dem die Kohlenwasserstoffe kondensiert werden. Die Hauptmenge der flüssigen Nichtaromaten wird anschließend über die Leitung 18 aus dem Verfahren abgezogen und seiner weiteren Verwendung zugeführt, während ein kleiner Teilstrom über die Leitung 19 als Rückfluß am Kopf in den Kolonnenteil 15 wiedereingeleitet wird. Die Rückflußmenge wird dabei so eingestellt, daß die gewonnenen Nichtaromaten den gewünschten Reinheitsgrad aufweisen. Überraschenderweise hat sich gezeigt, daß dies bei Anwendung des erfindungsgemäßen Verfahrens bereits in den allermeisten Fällen mit einem Rückflußverhältnis von 0,5 erreicht werden kann, während bisher normalerweise mit einem Rückflußverhältnis von 1 gearbeitet wurde.

Für die Beheizung ist am Sumpf der Extraktivdestillationskolonne 8 der Umlaufkocher 20 vorgesehen, in dem das umlaufende Sumpfprodukt im indirekten Wärmeaustausch mit Dampf erhitzt wird. Die Leitungen 21 und 22 dienen hierbei der Zu- und Abführung des Dampfes und die Leitungen 23 und 24 der Zu- und Abführung des Sumpfproduktes. Selbstverständlich können an der Extraktivdestillationskolonne 8 auch noch weitere Seitenkocher zur zusätzlichen Kolonnenbeheizung angeordnet sein. Da die Kolonnenbeheizung jedoch nicht Gegenstand der vorliegenden Erfindung ist, braucht auf diese Ausführungsformen nicht näher eingegangen zu werden.

Die Wirksamkeit des erfindungsgemäßen Verfahrens wird durch die Ergebnisse der nachfolgenden Versuchsreihe belegt. Als Einsatzkohlenwasserstoffgemisch wurde hierbei ein Pyrolysebenzin verwendet, wobei die Durchsatzkapazität der Anlage ca. 14 600 kg/h betrug. In Teil a) der Versuchsreihe (Versuch 1) wurde die Reinigung des Raffinates in der bisher üblichen Weise in einer separaten Destillationskolonne vorgenommen. Teil b) betrifft demgegenüber die Anwendung des erfindungsgemäßen Verfahrens, wobei bei Versuch 2 das erhitzte Einsatzkohlenwasserstoffgemisch direkt in die Extraktivdestillationskolonne eingeleitet wurde. Bei Versuch 3 wurde dagegen das erhitzte Einsatzkohlenwasserstoffgemisch zunächst in eine flüssige und eine dampfförmige Phase zerlegt und beide Phasen getrennt voneinander in die Extraktivdestillationskolonne eingeleitet. Die erzielten Versuchsergebnisse sind in der nachfolgenden Tabelle zusammengefaßt.

Tabelle

| Ver-such Nr. | Zulauf temperatur Einsatz-produkt °C | Zulaufboden (v.oben) Dampf | Flüssigkeit | Nicht-aromaten im Benzol ppm | Lösungsmittel i.Nicht-aromat. (Raffinat) ppm | Fremd-energ.-bedarf Gcal/h |
|---|---|---|---|---|---|---|
| a) Extraktivdestillation nach dem Stande der Technik ||||||| 
| 1 | 80 | - | 24 | 170 | $3 . 10^{-3}$ | 1.806 |
| b) Extraktivdestillation nach der Erfindung |||||||
| 2 | 130 | - | 24 | 123 | $< 3 . 10^{-3}$ | 1,803 |
| 3 | 130 | 30 | 20 | 98 | $< 3 . 10^{-3}$ | 1,803 |

Die vorliegenden Versuchsergebnisse zeigen eindeutig, daß bei Anwendung des erfindungsgemäßen Verfahrens trotz des Fortfalls einer separaten Raffinatdestillationskolonne die gleiche Reinheit der Kohlenwasserstoffe des Raffinats (Nichtaromaten) erzielt werden konnte. Gleichzeitig wurde eine deutlich verbesserte Reinheit des gewonnenen Benzols erreicht. Durch die erfindungsgemäße Vorerhitzung des Einsatzkohlenwasserstoffgemisches wurde außerdem erreicht, daß der Fremdenergiebedarf beim erfindungsgemäßen Verfahren sogar geringfügig niedriger war als beim Verfahren nach dem Stande der Technik. Durch den Fortfall einer separaten Raffinatdestillationskolonne ergibt sich schließlich eine deutliche Einsparung an Anlage- und Wartungskosten.

Die Vorteile des erfindungsgemäßen Verfahrens dürften somit klar auf der Hand liegen.

**Patentansprüche**

1. Verfahren zur Abtrennung von Aromaten aus Kohlenwasserstoffgemischen beliebigen Aromatengehaltes, die als nichtaromatische Bestandteile insbesondere Paraffine, Cycloparaffine, Olefine, Diolefine sowie organische Schwefelverbindungen enthalten können, durch Extraktivdestillation, bei der N-substituierte Morpholine, deren Substituenten nicht mehr als sieben C-Atome aufweisen, als selektives Lösungsmittel verwendet werden, wobei die nichtaromatischen Bestandteile des als Einsatzprodukt dienenden Kohlenwasserstoffgemisches als Raffinat über Kopf aus der Extraktivdestillationskolonne abdestilliert werden, während die Aromaten zusammen mit dem verwendeten Lösungsmittel als Extrakt aus dem Sumpf der Extraktivdestillationskolonne abgezogen und anschließend in einer nachgeschalteten Abtreiberkolonne destillativ vom Lösungsmittel abgetrennt werden, **dadurch gekennzeichnet**, daß sowohl die Extraktivdestillation als auch die Entfernung der Lösungsmittelreste aus dem Raffinat in einer einzigen gemeinsamen Kolonne durchgeführt werden, die oberhalb der Lösungsmittelaufgabe einen zusätzlichen Kolonnenteil aufweist, wobei das Einsatzkohlenwasserstoffgemisch vor dem Eintritt in diese Kolonne im indirekten Wärmeaustausch mit dem von der Abtreiberkolonne kommenden heißen Lösungsmittel bis auf eine Temperatur zwischen 130 und 150°C erhitzt wird.

2. Verfahren nach Anspruch 1, **dadurch gekennzeichnet**, daß das erhitzte Einsatzkohlenwasserstoffgemisch vor dem Eintritt in die Extraktivdestillationskolonne in eine flüssige und eine dampfförmige Phase zerlegt wird, die getrennt voneinander in diese Kolonne eingeleitet werden, wobei die Einleitungsstelle für die dampfförmige Phase unterhalb der Einleitungsstelle für die flüssige Phase liegt.

3. Verfahren nach den Ansprüchen 1 und 2, **dadurch gekennzeichnet**, daß die auf den Kopf des zusätzlichen Kolonnenteiles aufgegebene Rückflußmenge so eingestellt wird, daß das Rückflußverhältnis vorzugsweise bei 0,5 liegt.

**Claims**

1. Method of separating aromatics from hydrocarbon mixtures of arbitrary aromatics content, which can contain especially paraffins, cycloparaffins, olefins, diolefins and organic sulphur compounds as non-aromatic constituents, by extractive distillation in which N-substituted morpholines, whose substituents have no more than seven C atoms, are used as a selective solvent, the non-aromatic constituents of the hydrocarbon mixture used as feedstock being distilled off as raffinate at the top of the extractive distillation column, while the aromatics, together with the solvent used, are withdrawn as extract from the bottom of the extractive distillation column and are then separated from the solvent by distillation in a downstream stripping column, **characterised in that** both the extractive distillation and the removal of the solvent residues from the raffinate are carried out in a single common column having an additional column section above the solvent feed, the mixed hydrocarbon feedstock being heated to a temperature of between 130 and 150°C, before entering this column, by indirect heat exchange with the hot solvent coming from the stripping column.

2. Method according to Claim 1, **characterised in that**, before it enters the extractive distillation column, the heated mixed hydrocarbon feedstock is split into a liquid and a vapour phase, which are separately introduced into this column, the point at which the vapour phase is introduced being below the point at which the liquid phase is introduced.

3. Method according to Claims 1 and 2, **characterised in that** the quantity of reflux fed into the top of the additional column section is adjusted so that the reflux ratio is preferably 0.5.

**Revendications**

1. Procédé de séparation des fractions aromatiques à partir de mélanges d'hydrocarbures présentant des teneurs en fractions aromatiques quelconques pouvant contenir, à titre de composants non aromatiques, en particulier des paraffines, des cycloparaffines, des oléfines, des dioléfines, ainsi que des combinaisons soufrées organiques, par distillation extractive, dans lequel la morpholine substituée en N, dont les substituants ne présentent pas plus de 7 atomes de C, sont utilisés comme solvant sélectif, les composants non aromatiques du mélange d'hydrocarbures servant de produit de mise en oeuvre sont séparés par distillation, à titre de raffinat, à la tête de la colonne de distillation extractive, tandis que les fractions aromatiques sont extraites, conjointement avec les solvants utilisés, à titre d'extrait, du pied de la colonne de distillation extractive, puis séparées du solvant par voie de distillation, dans une colonne de rectification mise en circuit en aval,
caractérisé en ce que tant la distillation extractive qu'également l'élimination des résidus de solvants hors du raffinat sont effectuées dans une colonne commune unique, présentant, au dessus de l'alimentation en solvant, une partie de colonne supplémentaire, le mélange de mise en oeuvre à base d'hydrocarbures étant chauffé à une température située entre 130 et 150° C, avant entrée dans cette colonne, selon un échange calorifique indirect avec le solvant chaud venant de la colonne de rectification.

2.   Procédé selon la revendication 1,
caractérisé en ce que le mélange de mise en oeuvre à base d'hydrocarbures chauffé est décomposé, avant l'entrée dans la colonne de distillation extractive, en une phase liquide et une phase vapeur, introduites séparément l'une de l'autre dans cette colonne, le point d'introduction de la phase vapeur étant situé au dessous du point d'introduction de la phase liquide.

3.   Procédé selon les revendications 1 et 2,
caractérisé en ce que le débit recyclé introduit à la tête de la partie de colonne supplémentaire est réglé de telle façon que le taux de recyclage soit de préférence égal à 0,5.